# EUROPÄISCHE PATENTSCHRIFT

(11) **EP 2 206 705 B1**
(45) Veröffentlichungstag und Bekanntmachung des Hinweises auf die Patenterteilung: **13.03.2013**
(21) Anmeldenummer: 09178883.6
(22) Anmeldetag: 11.12.2009
(51) Int. Cl.: C07D 265/36

(54) **Verfahren zur Herstellung von Benzomorpholinderivaten durch Hydrierung von o-Nitrophenoxycarbonylverbindungen**
Method for manufacturing benzomorpholine derivatives through hydration of o-nitrophenoxycarbonyl compounds
Procédé de fabrication de dérivés de benzomorpholine par hydrogénisation de liaisons d'o-nitrophénoxycarbonyle

(30) Priorität: 23.12.2008 DE 102008062906
(43) Veröffentlichungstag der Anmeldung: 14.07.2010
(73) Patentinhaber: Saltigo GmbH, 40764 Langenfeld (DE)
(72) Erfinder: BOLL, Matthias, 51061 Köln (DE); KOCH, Burkhard, 50823 Köln (DE)
(74) Vertreter: Pettrich, Klaus-Günter

(56) Entgegenhaltungen:
- WO-A-2006/118923
- US-A- 2 381 935
- US-A- 4 623 650
- US-A- 4 638 067
- US-A1- 2004 044 206

## Beschreibung

Die Erfindung betrifft ein Verfahren zur Herstellung von Benzomorpholinderivaten, bei dem eine o-Nitrophenoxycarbonylverbindung in Gegenwart von mit Fremdmetallen dotierten Sponge-Metal-Katalysatoren auf der Basis von Nickel oder Kobalt unter Ringbildung hydriert wird..

Die Hydrierung von ortho-Nitrophenoxyaceton (oNPA) zu 3-Methylbenzomorpholin (3MBM) ist eine kommerziell verwendete chemische Umsetzung mit Wasserstoff unter Druck in Gegenwart eines Katalysators. Die Reaktion wird großtechnisch durch Edelmetallkatalysatoren, hier vor allem Pt-Katalysatoren auf Aktivkohle, katalysiert.

3-Methylbenzomorpholin (3MBM) ist ein technisch hergestelltes Zwischenprodukt für die Herstellung von Farbstoffen und in der Pflanzenschutzmittelindustrie. Die Herstellung von 3-MBM geschieht zurzeit durch die Umsetzung unter mäßigem Wasserstoffüberdruck an einem Edelmetallkatalysator. Nachteile dieses Verfahrens sind die niedrigen Standzeiten des Katalysators, die angegebene diskontinuierliche Fahrweise und die vergleichsweise langen Hydrierzeiten bei gleichzeitig hohen Kosten für den edelmetallhaltigen Katalysator. Zusätzlich wird eine Reihe von Nebenprodukten gebildet, die die Ausbeute und Reinheit des Produktes reduzieren. Dieses Verfahren wird in der US2006/0017946A1 beschrieben.

Anstelle der teuren, edelmetallhaltigen Katalysatoren könnten grundsätzlich auch sogenannte Sponge-Metal-Katalysatoren (auch Skelett-Katalysatoren genannt) auf Nickel-Basis eingesetzt werden, wie in der US 2381935 anhand eines Beispiels beschrieben. Ein solcher Katalysator wird durch Laugung einer Aluminium-Nickel-Legierung mit zum Beispiel Natronlauge hergestellt. Dort wurde unter vergleichsweise drastischen Bedingungen bei 120°C-170°C eine 56 Gew.-%ige Lösung von oNPA in Methanol mit einem nicht näher beschriebenen Raney-Nickel-Katalysator hydriert. Es wurden in dem angeführten Beispiel etwa 20g oNPA pro g Katalysator umgesetzt.

Die Reduktion von aromatischen Nitrogruppen mit Wasserstoff zu den entsprechenden Aminen als eine (in der vorliegenden Reaktion nicht isolierte und nur vermutete) Zwischenstufe wird in der chemischen Industrie weltweit großtechnisch durchgeführt. Grundsätzlich sind für Nitroreduktionen eine ganze Reihe von Katalysatoren geeignet, angefangen von Nickel- oder Kobaltkatalysatoren (in Form der entsprechenden "Sponge-Metal-Katalysatoren" oder auch fein verteilt auf Trägermaterialien). Gemische aus Nickel und anderen Metallen, wie zum Beispiel Eisen, Molybdän, Chrom (alle ebenfalls als "Sponge- Metal -Katalysatoren" bezeichnet) sind ebenfalls prinzipiell geeignet. Solche Katalysatoren werden durch Laugung einer aluminiumhaltigen Metalllegierung, die die entsprechenden Elemente enthält, in der Regel mit Alkalilauge bei erhöhten Temperaturen hergestellt. Diese Legierungen können auch bei niedriger Temperatur in wässriger Phase unter Zugabe von Ammoniumchlorid zur Nitroreduktion eingesetzt werden, wie Bhaumik (Canadian Journal of Chemistry (2003), 81(3), 197-198) berichtet.

Neben diesen vergleichsweise kostengünstigen Katalysatortypen kommen für die Nitroreduktion auch Edelmetallkatalysatoren auf Trägermaterialien in Frage, zum Beispiel Platin oder Palladium auf Aktivkohle, die wie die Sponge-Metal-Katalysatoren auch mit der Zugabe von Ammoniak oder einem anderen Amin oder einem andere Katalysatorgift gezielt in der Reaktivität gebremst werden können. Diese Katalysatoren haben dabei zwei Nachteile gegenüber den Sponge-Metal-Katalysatoren auf Nickel- oder Kobaltbasis: zum einen sind sie in der Regel schlechter filtrierbar und die damit verbundenen Katalysatorverluste sind größer, zum anderen sind die Anschaffungs- und Recyclingkosten für edelmetallhaltige Katalysatoren in der Regel deutlich höher.

Aus diesen Gründen werden in der Technik in der Regel sehr häufig Sponge-Metal -Katalysatoren eingesetzt.

Andere Möglichkeiten, Nitrogruppen zu reduzieren, wie etwa die im Organikum (21. Auflage, S. 627 ff., Wiley VCH, Weinheim) vorgeschlagene Reduktion mit Zink, Zinn oder Eisen in HCl, spielen in der chemischen Industrie ebenso eine nur untergeordnete Rolle wie die Reduktion mit zum Beispiel Hydrazin, wie sie unter anderem von Han et al. (Tetrahedron Letters (1985), 26(50), 6233-4) beschrieben wurde. Auch homogene Katalysatoren auf Basis von Edelmetallen wurden für die Reduktion bereits erwähnt, zum Beispiel durch Sandra et al. (Journal of Molecular Catalysis (1987), 39(3), 279-92). In den oben angegebenen Beispiele wurde außerdem immer nur die Nitrogruppe am Aromaten hydriert, eine weitere, intramolekular reduktive Aminierung wie bei der Herstellung von 3MBM vermutet, wurde hier nicht betrachtet.

Es gibt auch die Möglichkeit, zur Reduktion Metallkatalysatoren auf einem anorganischen Träger zu verwenden, wie etwa einen Kupferkatalysator in Gegenwart von Nickel und / oder Palladium auf einem Silikat (Patent: CN1861253). Ebenso wurde durch Lu et al. (Zhejiang Gongye Daxue Xuebao (2002), 30(5), 464-466) von Carbonanotubes als Trägermaterial berichtet.

Aufgrund der thermischen Instabilität von oNPA (in einer etwa 20 Gew.-%ige Lösung oNPA in einem Gemisch mit etwa 20 Gew.-% Toluol und etwa 60 Gew.-% Methanol beginnt bereits bei etwa 130°C eine deutliche Exothermie durch Zersetzung des Eduktes) und der zu erwartenden Wärmeentwicklung während der Reaktion sowie wegen der beobachteten geringen Umsätze von nur etwa 70% ist eine solche Reaktionsführung großtechnisch nicht ausführbar.

Aufgabe der vorliegenden Erfindung war es, ein Verfahren zur Herstellung von Benzomorpholinderivaten durch Hydrierung von o-Nitrophenoxycarbonylverbindungen zur Verfügung zu stellen, bei dem die Hydrierung dieser Nitrophenoxycarbonylverbindungen bei einer möglichst niedrigen Temperatur und in Gegenwart eines kostengünstigen Katalysators durchgeführt wird.

Es wurde nun das erfindungsgemäße Verfahren gefunden, das diese Aufgabe löst.

Gegenstand der Erfindung ist daher ein Verfahren zur Herstellung von Benzomorpholinderivaten der allgemeinen Formel (I) 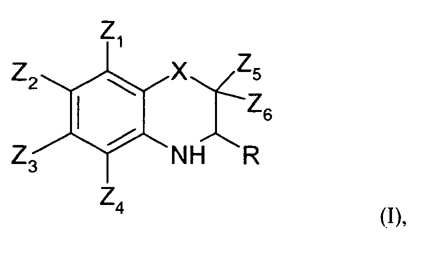 in der
- R: Wasserstoff, ein C₁-C₁₀-Alkyl-, Phenyl- oder Tetrahydrofurfurylrest sein kann,
- Z₁ bis Z₆: jeweils unabhängig Wasserstoff oder ein C₁-C₁₀-Alkylrest und
- X: Sauerstoff oder Schwefel sein kann,
durch Hydrierung von Nitrophenoxycarbonylverbindungen der allgemeinen Formel (II) 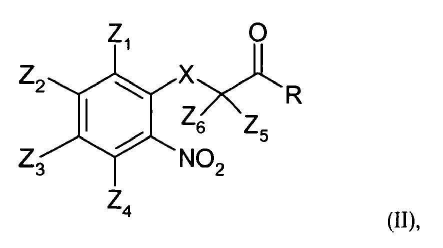 in der
R, Z₁ bis Z₆ und X die für Formel (I) angegebene Bedeutung haben,
**dadurch gekennzeichnet, dass** die Hydrierung von Verbindungen der allgemeinen Formel (II) unter Ringbildung mit Wasserstoff-Gas in Gegenwart eines mit Molybdän und/oder Eisen und/oder Aluminium dotierten Sponge-Metal-Katalysators auf der Basis von Nickel oder Kobalt durchgeführt wird und der pH-Wert während der Hydrierung auf einen Wert zwischen 7 und 10 eingestellt wird.

Für die Hydrierung der o-Nitrophenoxycarbonylverbindung konnte überraschenderweise gezeigt werden, dass sich diese Reaktion in Gegenwart von Sponge-Metal-Katalysatoren auf Basis einer Nickel/Molybdän-Legierung praktisch ohne wesentliche Nebenreaktionen (zum Beispiel zu Dimeren des 3-Methyl-Benzomorpholin oder zu teilhydrierten oder kernhydrierten Verbindungen oder zu anderen, unerwünschten Nebenprodukten) bei niedrigen Temperaturen (30-90°C) und niedrigen Verweilzeiten (etwa 1 Stunde) im technischen Maßstab durchführen lässt.

Sponge-Metal-Katalysatoren auf Basis von undotiertem Kobalt sowie auf Basis von undotiertem Nickel sind für die Umsetzung bei niedrigen Temperaturen völlig ungeeignet, während sich ein Molybdän dotierter nickelhaltiger Katalysator als besonders günstig herausstellte und ein mit dem edelmetallhaltigen Katalysator vergleichbares Ergebnis zeigte. Bevorzugt wird ein mit Molybdän dotierter Katalysator, z.B. mit ein Sponge-Metall-Katalysator auf Nickel-Basis, der neben Nickel und Aluminium noch zwischen 0,5 und 3% Molybdän enthält, wie er z.B. unter dem Handelsnamen AMPERKAT Ni-Mo 3706 von der Fa. H.C. Starck (Goslar, Germany) erhältlich ist, eingesetzt. Für die Herstellung des Katalysators muss bereits in der Vorlegierung Molybdän enthalten sein. Die Vorlegierung wird geschmolzen, dann durch Verdüsen in Wasser sehr rasch abgekühlt und anschließend mit Natronlauge gelaugt. Neben dem molybdänhaltigen Katalysatoren können auch eisenhaltige Sponge-Metall-Katalysatoren auf Nickelbasis mit einem Eisengehalt von 12-15% Eisen im fertigen Katalysator verwendet werden (zum Beispiel der AMPERKAT Ni-Fe 6606 von der Fa. H.C. Starck (Goslar, Germany), der aus einer eisenhaltigen Nickel-Aluminium-Vorlegierung, durch Verdüsen in Wasser hergestellt werden kann). Außerdem kommt als Dotierungselement auch Chrom in Betracht, vor allem in Kombination mit Eisen, Nickel und Aluminium und in Form einer langsam erstarrten Vorlegierung, die anschließend zerkleinert und gelaugt wird (zum Beispiel der AMPERKAT Ni-FeCr 4546 von der Fa. H.C. Starck (Goslar, Germany)).

Außerdem zeigt der im erfindungsgemäßen Verfahren eingesetzte Sponge-Metal- Katalysator unter den gewählten Versuchsbedingungen mit >90g Nitroverbindung pro Gramm Katalysator (ohne Zwischenwäsche des Katalysators) überraschend eine deutlich höhere Standzeit als ein edelmetallhaltiger Katalysator.

Im allgemeinen führt man die Hydrierung in einem Lösungsmittelgemisch aus einem C₁-C₃-Alkohol wie Methanol, Ethanol oder iso-Propanol im Gemisch mit einen aromatischen Lösungsmittel wie Toluol aus, oder auch ausschließlich in dem entsprechenden Alkohol, sofern die Löslichkeit des oNPa bei den vorhandenen niedrigen Temperaturen ausreicht. Die Mischungsverhältnisse (in Gewichtsanteilen) liegen zwischen 1:100 aromatisches Lösungsmittel zu Alkohol bis zu 20:100 aromatisches Lösungsmittel zu Alkohol, so dass auch unter der Berücksichtigung des entstandenen Reaktionswasser bevorzugt (aber nicht notwendigerweise) eine Einphasigkeit des Reaktionsgemisches am Ende der Reaktion gewährleistet bleibt. Bevorzugt ist ein Toluol/Methanol - Gemisch als Lösungsmittel für die Hydrierung mit einem Mischungsverhältnis von 9,7:100 Toluol zu Methanol. In Anlehnung an die Beschreibung aus US2006/0017946A1 können aber auch Lösungsmittelgemische mit einem deutlich höheren Anteil an Toluol verwendet werden.

Der Einfluss des im Lösungsmittelgemisch gemessenen "pH-Wertes" (der Begriff pH-Wert könnte im vorliegenden Fall eigentlich durch eine gemessene Spannung gegen eine Referenzelektrode ersetzt werden, da in einem nicht-wässrigen System gemessen wurde) hat einen überraschenden Einfluss auf die Bildung von Nebenkomponenten und die Standzeit des Katalysators. Als günstig hat sich ein pH-Wert zwischen 7 und 10, vorzugsweise zwischen 8 und 10, besonders bevorzugt 8-9, erwiesen. Üblicherweise wird der pH-Wert dabei mit einer handelsüblichen pH-Glaselektrode gemessen.

Der Wasserstoffdruck liegt während der Hydrierung üblicherweise zwischen 10 und 400 bar, bevorzugt zwischen 200 und 240 bar, besonders bevorzugt bei 220 bar.

Die Reaktionstemperatur kann bei der Hydrierung üblicherweise im Bereich zwischen 30 und 90°C, bevorzugt bei 60°C, liegen.

Die Hydrierung kann diskontinuierlich oder bevorzugt kontinuierlich ausgeführt werden. Z. B. kann in einer kontinuierlichen Verfahrensweise die Verweilzeit der Eduktlösung zwischen 30 und 180 Minuten, bevorzugt 60 Minuten betragen und die Katalysatorbelastung im Bereich von 0,1 bis 20 g, bevorzugt 1,5 bis 3g, Nitroverbindung pro Gramm Katalysator und Stunde liegen.

Durch Wahl dieser Versuchsparameter konnte die Anzahl und Menge der Nebenkomponenten minimiert werden. Besonders relevant ist hier unter anderem die Bildung des 3-MBM-Dimeren, welche sonst bei der Wahl falscher Reaktionsbedingungen ansteigt und die Ausbeute und Reinheit an 3-MBM spürbar verringert.

Nach dem erfindungsgemäßen Verfahren können allgemein Benzomorpholinderivate der angegebenen Formel (I) hergestellt werden. Hierunter werden solcher verstanden, bei denen X ein Sauerstoffatom ist (Benzomorpholinderivate), aber auch solche verstanden, bei denen X ein Schwefelatom ist (Benzoparathiazinderivate). Bevorzugt ist X ein Sauerstoffatom.

Besonders bevorzugt wird nach dem erfindungsgemäßen Verfahren o-Nitrophenoxyaceton, das z.B. aus der Umsetzung von o-Nitrophenol mit Chloraceton hergestellt werden kann, zum 3,4-Dihydro-3-methyl-2H-1,4-benzoxazin (3-MBM) hydriert (Z₁-Z₆ = Wasserstoff, X = Sauerstoff und R = Methyl in Formel (I)). 3-MBM kann z.B. zum 4-(Dichloracetyl)-3,4-dihydro-3-methyl-H-1,4-benzoxazin (Benoxacor) weiterverarbeitet werden, das als Safener in Herbiziden eingesetzt wird.

Die folgenden Beispiele sollen die Erfindung weiter erläutern, ohne sie jedoch in ihrem Umfang zu begrenzen.

### Beispiele

Bei den beschriebenen Beispielen 1, 2 und 11 wurde immer von reinem, kristallisiertem o-NPA ausgegangen, das in Anlehnung an die Vorschrift aus US2006/0017946A1 abschließend durch Kristallisation aus einem isopropanolhaltigen Lösungsmittelgemisch hergestellt wurde.

Folgende Katalysatoren wurden in den Beispielen verwendet (alle Angaben in Gew.-%) :
- Katalysator 1:: Amperkat SK Co 3706 der Firma HC Starck, Goslar, aus verdüster und damit rascherstarrter Vorlegierung, die im wesentlichen Kobalt und Aluminium enthält. Der eingesetzte Katalysator enthält 94-98% Kobalt, 1-5% Aluminium, maximal 1,5% Nickel, maximal 1% Eisen
- Katalysator 2:: Amperkat SK Ni-Mo 3706 der Firma HC Starck, hergestellt durch Laugung einer verdüsten (rascherstarrten) Vorlegierung, die Nickel, Aluminium und Molybdän enthält. Der eingesetzte Katalysator enthält 91-96% Nickel, 4-7% Aluminium, 0,5- 3% Molybdän sowie maximal 1% Eisen
- Katalysator 3:: Amperkat SK Ni 3706 der Firma HC Starck, Goslar, hergestellt durch Laugung einer verdüsten (rascherstarrten) Vorlegierung, die Nickel und Aluminium enthält. Der eingesetzte Katalysator enthält 93-96% Nickel, 3-7% Aluminium sowie maximal 1% Eisen
- Katalysator 4:: Amperkat SK Ni-Fe 6606 der Firma HC Starck, Goslar, hergestellt durch Laugung einer verdüsten (rascherstarrten) Vorlegierung, die Nickel, Eisen und Aluminium enthält. Der eingesetzte Katalysator enthält 77-82% Nickel, 4-7% Aluminium sowie 12-15% Eisen
- Katalysator 5:: Amperkat SK Ni-FeCr 4546 der Firma HC Starck, Goslar, hergestellt durch Laugung einer in Barren (langsam erstarrten) Vorlegierung, die Nickel, Eisen, Chrom und Aluminium enthält. Der eingesetzte Katalysator enthält 82-90% Nickel, 6-10% Aluminium, 2-5% Chrom sowie 2-5% Eisen
- Katalysator 6:: K-0126 10% Pt-Metall auf Aktivkohle der Firma Heraeus, Hanau (Vergleich)
- Katalysator 7:: K-8610 1% Pt-Metall auf Kohle der Firma Heraeus, Hanau (Vergleich)
- Katalysator 8:: 5% Pd-Metall auf Aktivkohle (Vergleich)
- Katalysator 9:: hergestellt aus einer Vorlegierung nach Vorschrift aus der Patentanmeldung DE197 53501A1, Beispiel 3 und gelaugt in Natronlauge.

Bei den Angaben zu den Gehalten von 3MBM handelt es sich um Gew.-% Angaben, die aus kalibrierten GC-Messungen erhalten wurden. Trotzdem sind diese Angaben mit Unsicherheiten behaftet, so dass auch Messwerte geringfügig höher als 100% entstehen können. Der angegebene Gehalt an 3MBM-Dimeren wurde nicht kalibriert (bei den angegebenen Messwerte handelt es sich um Flächenprozente der GC-Methode), so dass diese Angaben nur als Richtwert und im relativen Vergleich verstanden werden können.

### Beispiel 1

### Vergleich verschiedener "Sponge-Metal -Katalysatoren" und eines Edelmetallkatalysators bei der Hydrierung von o-Nitrophenoxyaceton zu 3MBM in Isopropanol/Toluol bei 60°C / 100bar Wasserstoffdruck

In sechs identischen Hochdruckreaktoren wurden jeweils 1,3 g o-Nitrophenoxyaceton (mit nahezu 100%iger Reinheit laut GC-Analyse), 1,7g Iso-Propanol (zur Analyse), und 6,9g Toluol zusammen mit 0,6g Katalysator (bezogen auf das Trockengewicht) eingewogen.

Es wurde ein Wasserstoffdruck von 100bar eingestellt und die Temperatur auf 40°C erhöht und für 30 Minuten unter diesen Bedingungen belassen. Dann wurde der Druck in den Reaktoren auf Umgebungsdruck erniedrigt und das Reaktionsprodukt per GC-Analytik untersucht.

| Katalysator | Gehalt 3MBM (Gew.-%) | Gehalt oNPA (Gew.-%) | Anzahl Nebenkomponenten | Summe Nebenkomponenten (Flächen-%) |
|---|---|---|---|---|
| 1 | 0 | 7,0 | 3 | 1,5 |
| 2 | 9,1 | 0 | 1 | 1,7 |
| 3 | 1,4 | 0 | 2 | 0,9 |
| 4 | 2,8 | 0 | 1 | 2,5 |
| 5 | 7,2 | 0 | 1 | 2,8 |
| 6 (Vergleich) | 8,7 | 0 | 0 | 0 |

### Beispiel 2

### Vergleich verschiedener "sponge metal Katalysatoren" und eines Edelmetallkatalysators bei der Hydrierung von o-Nitrophenoxyaceton zu 3MBM in Isopropanol/Toluol bei 85°C / 200bar Wasserstoffdruck

In sechs identischen Hochdruckreaktoren wurden jeweils 8 g einer 16,4 Gew.-% o-Nitrophenoxyaceton-Lösung (gelöst in einem Gemisch aus 55,6 Gew.-% Methanol und 28 Gew.-% Toluol), zusammen mit 0,6g Katalysator (bezogen auf das Trockengewicht) eingewogen.

Es wurde ein Wasserstoffdruck von 200bar eingestellt und die Temperatur auf 85°C erhöht und für 15 Minuten unter diesen Bedingungen belassen. Dann wurde der Druck in den Reaktoren auf Umgebungsdruck erniedrigt und das Reaktionsprodukt per GC-Analytik untersucht.

| Katalysator | Gehalt 3MBM (Gew.-%) | Gehalt oNPA (Gew.-%) | Anzahl Nebenkomponenten | Summe Nebenkomponenten (Flächen-%) |
|---|---|---|---|---|
| 5 | 6,5 | 0,1 | 12 | 3,4 |
| 7 | 9,2 | 0 | 8 | 2,4 |
| 1 | 9,6 | 0 | 8 | 1,6 |
| 4 | 8,8 | 0,2 | 8 | 2,1 |
| 8 (Vergleich) | 9,5 | 0 | 9 | 3,0 |
| 9 | 7,4 | 0,2 | 11 | 2,4 |

### Beispiel 3

### Kontinuierliche Hydrierung einer 20,6 Gew.-%iger o-NPA-Lösung mit Hilfe eines Hochdruckautoklaven (mit Überlauf) bei 150 bar Wasserstoff unter Verwendung des Katalysators 2 bei Temperaturen zwischen 70-85°C

In einem Autoklaven aus Edelstahl, der mit einer Fritte an einen Überlauf angeschlossen wurde (so dass das innere, für Flüssigkeiten verfügbare Volumen auf etwa 455 ml begrenzt wurde), wurden 15g Katalysator 2 (gerechnet als getrocknete Ware) in 200g Methanol vorgelegt. In diese gerührte Suspension wurde unter einem Wasserstoffdruck von 150 bar eine etwa 20 Gew.-%iger o-NPA-Lösung (mit 20 Gew.-% Toluol und 60 Gew.-% Methanol, hergestellt nach dem in US2006/0017946A1 beschriebenen Verfahren ohne weitere Aufreinigung) eindosiert, so dass die anfängliche mittlere Verweilzeit der Lösung bei etwa 60 Minuten lag. Im Verlauf des Versuches wurde die Temperatur erhöht und die Verweilzeit verlängert. In zeitlichen Abständen von 1 bis 2 Stunden wurden Proben gezogen und gemessen.

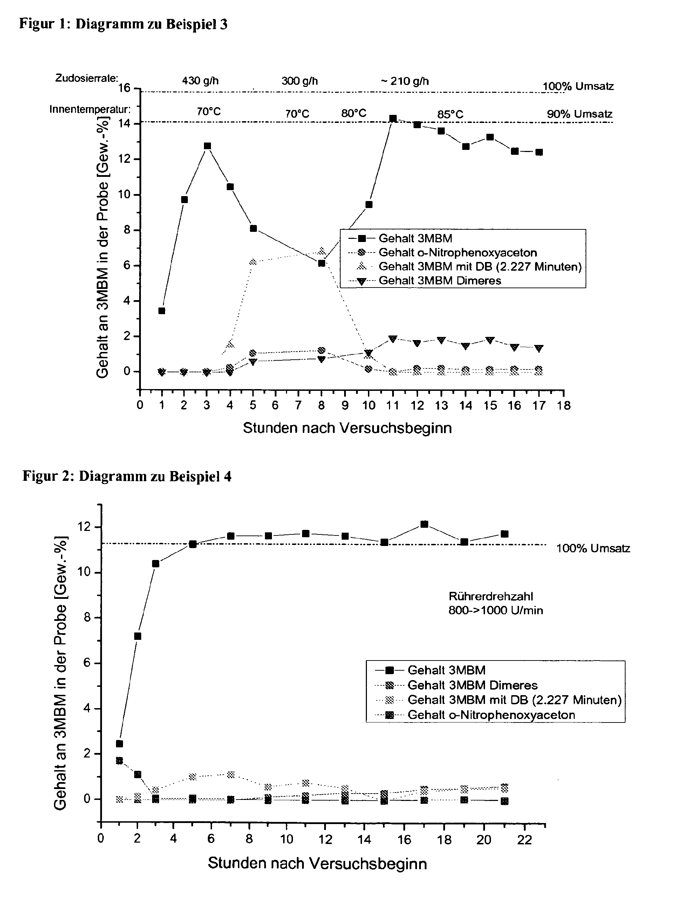

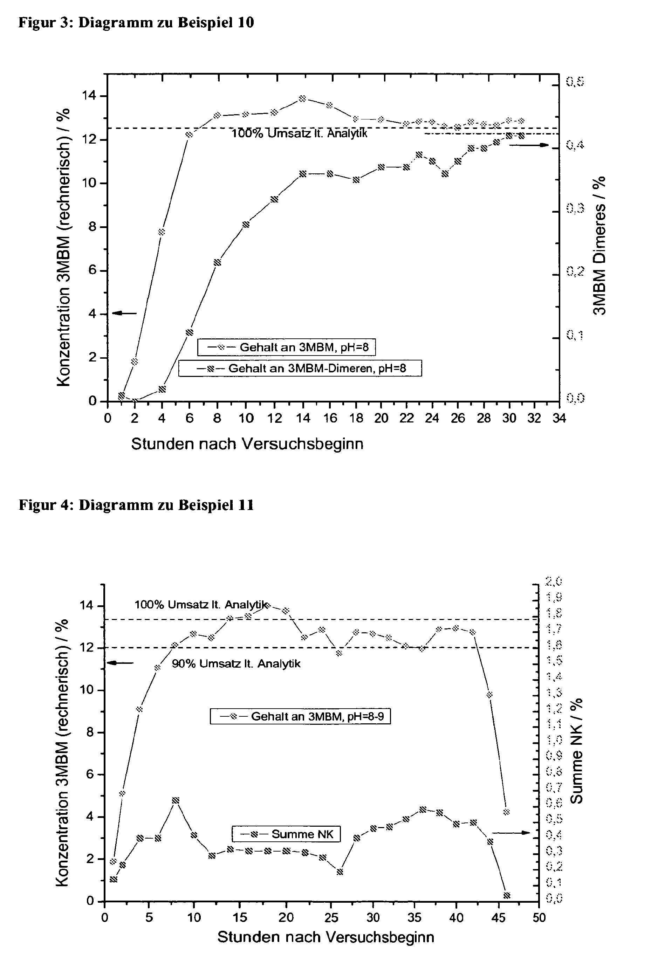

Die Ausbeute an 3MBM lag bei allen Versuchsparametern fast immer deutlich unterhalb der wirtschaftlich notwendigen 90% (siehe Figur 1: Diagramm zu Beispiel 3), der Gehalt an Nebenkomponenten war vergleichsweise hoch. Dennoch kann der Katalysator unter diesen Bedingungen in einem kontinuierlichen Prozess als Hydrierkatalysator verwendet werden und es konnte ein Umsatz von 66g o-Nitrophenoxyaceton pro Gramm Katalysator realisiert werden. Der Gesamtumsatz zu 3MBM lag bei 78 % und es wurden 11% 3MBM-Dimeres bezogen auf das gebildete 3MBM ermittelt.

### Beispiel 4

### Kontinuierliche Hydrierung einer 14,8 Gew.-%igen o-NPA-Lösung mit Hilfe eines Hochdruckautoklaven (mit Überlauf) bei 200 bar Wasserstoff unter Verwendung des Katalysators 2 bei einer Temperatur von 85°C

In einem Versuchsaufbau wie unter Beispiel 3 beschrieben wurden 45g Katalysator 2 (gerechnet als getrocknete Ware) in 200g Methanol vorgelegt. In diese gerührte Suspension wurde unter einem Druck von 200 bar Wasserstoff die oNPA-Lösung aus Beispiel 3 (mit Methanol verdünnt auf eine Konzentration von etwa 14,8 Gew.-%iger o-NPA) eindosiert, so dass die mittlere Verweilzeit bei etwa 1 Stunde lag. In Abständen von ein bis zwei Stunden wurden Proben gezogen und per GC-Analytik gemessen, Ergebnisse siehe Figur. Die analytische Ausbeute an 3MBM war über den Versuchszeitraum fast immer in der Nähe der rechnerischen Ausbeute. Der Gehalt an 3-MBM-Dimerem allerdings lag mit 5% bezogen auf das 3MBM recht hoch. Die in der Gesamtprobe gemessene Ausbeute an 3MBM lag über 90%.

Dennoch kann der Katalysator unter diesen Bedingungen in einem kontinuierlichen Prozess als Hydrierkatalysator verwendet werden. Es konnte im vorliegenden Versuch ein Umsatz von 40g o-Nitrophenoxyaceton pro Gramm Katalysator realisiert werden.

### Beispiel 5

### Kontinuierliche Hydrierung einer 16,2 Gew.-%iger o-NPA-Lösung mit Hilfe eines Hochdruckautoklaven (mit Überlauf) bei 200 bar Wasserstoff unter Verwendung des Katalysators 5 bei einer Temperatur von 85°C

In einem Versuchsaufbau wie unter Beispiel 3 beschrieben wurden 27g Katalysator 5 (gerechnet als getrocknete Ware) in 200g Methanol vorgelegt. In diese gerührte Suspension wurde unter einem Druck von 200 bar Wasserstoff die oNPA-Lösung aus Beispiel 3 (mit Methanol auf einen Gehalt von 16,2 Gew.-% verdünnt) so schnell eindosiert, dass die mittlere Verweilzeit bei etwa einer Stunde lag.

Die in der Gesamtprobe gemessene Ausbeute an 3MBM lag bei 81 % bezogen auf das eingesetzte o-Nitrophenoxyaceton. Der Gehalt an 3-MBM-Dimerem allerdings lag mit rund 10% bezogen auf das 3MBM zu hoch.

Dennoch kann der Katalysator unter diesen Bedingungen in einem kontinuierlichen Prozess als Hydrierkatalysator verwendet werden. Es konnte im vorliegenden Versuch ein Umsatz von 30g o-Nitrophenoxyaceton pro Gramm Katalysator realisiert werden.

### Beispiel 6

### Kontinuierliche Hydrierung einer 16,5 Gew.-%iger o-NPA-Lösung mit Hilfe eines Hochdruckautoklaven (mit Überlauf) bei 200 bar Wasserstoff unter Verwendung des Katalysators 5 bei einer Temperatur von 85°C

In einem Versuchsaufbau wie unter Beispiel 3 beschrieben wurden 40g Katalysator 5 (gerechnet als getrocknete Ware) in 200g Methanol vorgelegt. In diese gerührte Suspension wurde unter einem Wasserstoffdruck von 200 bar die oNPA-Lösung aus Beispiel 3 (mit Methanol auf einen Gehalt von 16,5 Gew.-% verdünnt) so schnell eindosiert, so dass die mittlere Verweilzeit bei 40 Minuten lag.

Die in der Gesamtprobe gemessene Ausbeute an 3MBM lag bei 97% bezogen auf das eingesetzte o-Nitrophenoxyaceton. Der Gehalt an 3-MBM-Dimerem allerdings lag mit rund 3,6% bezogen auf das 3MBM zu hoch.

Dennoch kann der Katalysator unter diesen Bedingungen in einem kontinuierlichen Prozess als Hydrierkatalysator verwendet werden. Es konnte im vorliegenden Versuch ein Umsatz von 12g o-Nitrophenoxyaceton pro Gramm Katalysator realisiert werden.

### Beispiel 7

### Kontinuierliche Hydrierung einer 16,5 Gew.-%iger o-NPA-Lösung mit Hilfe eines Hochdruckautoklaven (mit Überlauf) bei 200 bar Wasserstoff unter Verwendung des Katalysators 5 bei einer Temperatur von 60°C

In einem Versuchsaufbau wie unter Beispiel 3 beschrieben wurden 27g Katalysator 5 (gerechnet als getrocknete Ware) in 200g Methanol vorgelegt. In diese gerührte Suspension wurde unter einem Wasserstoffdruck von 200 bar die oNPA-Lösung aus Beispiel 3 (mit Methanol auf einen Gehalt von 16,5 Gew.-% verdünnt) so schnell eindosiert, so dass die mittlere Verweilzeit bei 55 Minuten lag.

Die in der Gesamtprobe gemessene Ausbeute an 3MBM lag bei 82% bezogen auf das eingesetzte o-Nitrophenoxyaceton. Der Gehalt an 3-MBM-Dimerem allerdings lag mit rund 3,3% bezogen auf das 3MBM zu hoch.

Dennoch kann der Katalysator unter diesen Bedingungen in einem kontinuierlichen Prozess als Hydrierkatalysator verwendet werden. Es konnte im vorliegenden Versuch ein Umsatz von 36g o-Nitrophenoxyaceton pro Gramm Katalysator realisiert werden.

### Beispiel 8

### Kontinuierliche Hydrierung einer 16,4 Gew.-%iger o-NPA-Lösung mit Hilfe eines Hochdruckautoklaven (mit Überlauf) bei 220 bar Wasserstoff unter Verwendung des Katalysators 2 bei einer Temperatur von 60°C

In einem Versuchsaufbau wie unter Beispiel 3 beschrieben wurden 25g Katalysator 2 (gerechnet als getrocknete Ware) in 200g Methanol vorgelegt. In diese gerührte Suspension wurde unter einem Druck von 220 bar Wasserstoff die oNPA-Lösung aus Beispiel 3 (mit Methanol auf einen Gehalt von 16,4 Gew.-% verdünnt) so schnell eindosiert, so dass die mittlere Verweilzeit bei 66 Minuten lag.

Die in der Gesamtprobe gemessene Ausbeute an 3MBM lag bei 74% bezogen auf das eingesetzte o-Nitrophenoxyaceton. 3-MBM-Dimeres konnte in den Proben nicht nachgewiesen werden.

Dennoch kann der Katalysator unter diesen Bedingungen in einem kontinuierlichen Prozess als Hydrierkatalysator verwendet werden. Es konnte im vorliegenden Versuch ein Umsatz von über 40g o-Nitrophenoxyaceton pro Gramm Katalysator realisiert werden.

### Beispiel 9

### Kontinuierliche Hydrierung einer 16,4 Gew.-%iger o-NPA-Lösung mit Hilfe eines Hochdruckautoklaven (mit Überlauf) bei 220 bar Wasserstoff unter Verwendung des Katalysators 2 bei einer Temperatur von 70°C

In einem Versuchsaufbau wie unter Beispiel 3 beschrieben wurden 25g Katalysator 2 (gerechnet als getrocknete Ware) in 200g Methanol vorgelegt. In diese gerührte Suspension wurde unter einem Druck von 220 bar Wasserstoff die oNPA-Lösung aus Beispiel 3 (mit Methanol auf einen Gehalt von 16,4 Gew.-% verdünnt) so schnell eindosiert, so dass die mittlere Verweilzeit bei 66 Minuten lag.

Die in der Gesamtprobe gemessene Ausbeute an 3MBM lag bei 96% bezogen auf das eingesetzte o-Nitrophenoxyaceton. Allerdings wurde 3-MBM-Dimeres zu 3,3 % bezogen auf das gebildete 3MBM nachgewiesen.

Dennoch kann der Katalysator unter diesen Bedingungen in einem kontinuierlichen Prozess als Hydrierkatalysator verwendet werden. Es konnte im vorliegenden Versuch ein Umsatz von etwa 40g o-Nitrophenoxyaceton pro Gramm Katalysator realisiert werden.

### Beispiel 10

### Kontinuierliche Hydrierung einer 16,4 Gew.-%iger o-NPA-Lösung mit Hilfe eines Hochdruckautoklaven (mit Überlauf) bei 220 bar Wasserstoff unter Verwendung des Katalysators 2 bei einer Temperatur von 60°C

In einem Versuchsaufbau wie unter Beispiel 3 beschrieben wurden 25g Katalysator 2 (gerechnet als getrocknete Ware) in 200g Methanol vorgelegt. In diese gerührte Suspension wurde unter einem Druck von 220 bar Wasserstoff die oNPA-Lösung aus Beispiel 3 (mit Methanol auf einen Gehalt von 16,4 Gew.-% verdünnt und mit NaOH auf einen pH-Wert von 8 eingestellt) so schnell eindosiert, so dass die mittlere Verweilzeit bei 66 Minuten lag.

Die in der Gesamtprobe gemessene Ausbeute an 3MBM lag bei über 90% bezogen auf das eingesetzte o-Nitrophenoxyaceton. Allerdings wurde 3-MBM-Dimeres zu 3,1 % bezogen auf das gebildete 3MBM nachgewiesen. Die Einzelwerte sind in Figur aufgetragen.

Dennoch kann der Katalysator unter diesen Bedingungen in einem kontinuierlichen Prozess als Hydrierkatalysator verwendet werden. Es konnte im vorliegenden Versuch ein Umsatz von 62g o-Nitrophenoxyaceton pro Gramm Katalysator realisiert werden.

### Beispiel 11

### Kontinuierliche Hydrierung einer 17,5 Gew.-%igen o-NPA-Lösung mit Hilfe eines Hochdruckautoklaven (mit Überlauf) bei 220 bar Wasserstoff unter Verwendung des Katalysators 2 bei einer Temperatur von 60°C

In einem Versuchsaufbau wie unter Beispiel 3 beschrieben, allerdings in einem Autoklaven mit einem Volumen von 900ml, wurden 50g Katalysator 2 (gerechnet als getrocknete Ware) in 900ml Methanol vorgelegt. In diese gerührte Suspension wurde unter einem Druck von 220 bar Wasserstoff eine 17,5 Gew.-%ige oNPA-Lösung (hergestellt aus kristallisisertem oNPA mit einem Gehalt >99% und 6,7 Gew.-% Toluol und 75,7 Gew.-% Methanol und gelagert bei 40°C und mit NaOH auf einem pH-Wert zwischen 8 und 9 eingestellt) so schnell eindosiert, so dass die mittlere Verweilzeit bei etwa 60 Minuten lag.

Die in der Gesamtprobe gemessene Ausbeute an 3MBM war nahezu quantitativ. Gebildete Nebenkomponenten lagen an der Nachweisgrenze. Details siehe Figur.

Es konnte im vorliegenden Versuch ein Umsatz von 92g o-Nitrophenoxyaceton pro Gramm Katalysator realisiert werden.

Dieses Beispiel wurde in fünf Versuchen reproduziert.

## Patentansprüche

1. Verfahren zur Herstellung von Benzomorpholinderivaten der allgemeinen Formel (I) 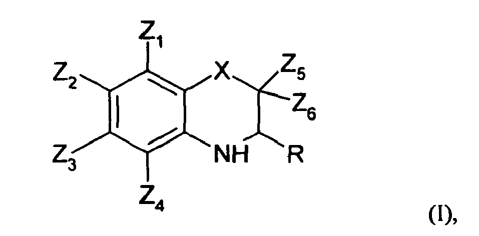 in der
R Wasserstoff, ein C₁-C₁₀-Alkyl-, Phenyl- oder Tetrahydrofurfurylrest ist,
Z₁, bis Z₆ jeweils unabhängig Wasserstoff oder ein C₁-C₁₀-Alkylrest und
X Sauerstoff- oder Schwefel ist,
durch Hydrierung von o-Nitrophenoxycarbonylverbindungen der allgemeinen Formel (II) 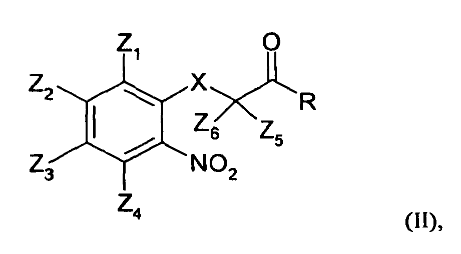 in der
R, Z₁ bis Z₆ und X die für Formel (I) angegebene Bedeutung haben,
**dadurch gekennzeichnet, dass** die Hydrierung von Verbindungen der allgemeinen Formel (II) mit Wasserstoff-Gas in Gegenwart eines mit Molybdän und/oder Eisen und/oder Aluminium und/oder Chrom dotierten Sponge-Metal-Katalysators auf der Basis von Nickel oder Kobalt durchgeführt wird und der pH-Wert während der Hydrierung auf einen Wert zwischen 7 und 10 eingestellt wird.

2. Verfahren nach Anspruch 1, **dadurch gekennzeichnet, dass** die o-Nitrophenoxycarbonylverbindung der allgemeinen Formel (II) vor der Hydrierung durch Umkristallisation gereinigt wurde.

3. Verfahren nach Anspruch 1 oder 2, **dadurch gekennzeichnet, dass** der Druck des Wasserstoff-Gases im Bereich zwischen 10 und 400 bar liegt.

4. Verfahren nach einem der Ansprüche 1 bis 3, **dadurch gekennzeichnet, dass**, die Temperatur während der Hydrierung im Bereich zwischen 40 und 90°C liegt.

5. Verfahren nach einem der Ansprüche 1 bis 4, **dadurch gekennzeichnet, dass** die Hydrierung kontinuierlich mit einer mittleren Verweilzeit der Eduktlösung in der Katalysatorsuspension zwischen 30 und 180 Minuten und einer Katalysatorbelastung von 0,1 bis 20 g Nitroverbindung pro Gramm Katalysator und Stunde durchgeführt wird.

6. Verfahren nach einem der Ansprüche 1 bis 5, **dadurch gekennzeichnet, dass** die Hydrierung in einem Lösungsmittelgemisch aus einem C₁-C₃-Alkohol und einem aromatischen Lösungsmittel durchgeführt wird.

7. Verfahren nach einem der Ansprüche 1 bis 6, **dadurch gekennzeichnet, dass** der Sponge-Metal-Katalysator ein Sponge-Metal-Katalysator auf der Basis von Nickel mit 0-15 Gew.-% Eisen ist.

8. Verfahren nach einem der Ansprüche 1 bis 6, **dadurch gekennzeichnet, dass** der Sponge-Metal-Katalysator ein Sponge-Metal-Katalysator auf der Basis von Nickel mit 0-4 Gew.-% Molybdän ist.

9. Verfahren nach einem der Ansprüche 1 bis 6, **dadurch gekennzeichnet, dass** der Sponge-Metal-Katalysator ein Sponge-Metal-Katalysator auf der Basis von Kobalt mit 0-5 Gew.-% Aluminium ist.

## Claims

1. Process for preparing benzomorpholine derivatives of the general formula (I) 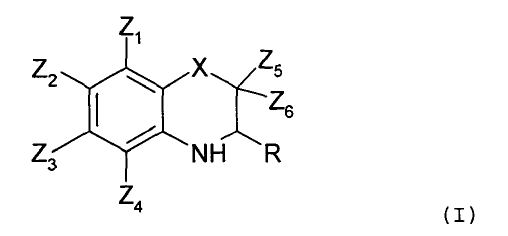 in which
R is hydrogen, or a C₁-C₁₀-alkyl, phenyl or tetrahydrofurfuryl radical,
Z₁ to Z₆ are each independently hydrogen or a C₁-C₁₀-alkyl radical and
X is oxygen or sulphur,
by hydrogenating o-nitrophenoxy carbonyl compounds of the general formula (II) 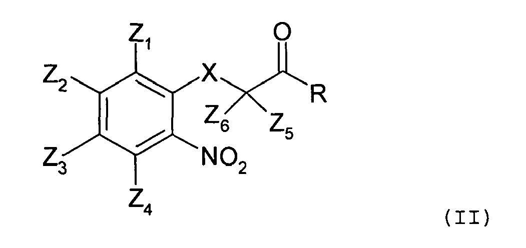 in which
R, Z₁ to Z₆ and X are each as defined for formula (I),
**characterized in that** the hydrogenation of compounds of the general formula (II) is performed with hydrogen gas in the presence of a molybdenum- and/or iron- and/or aluminium- and/or chromium-doped sponge metal catalyst based on nickel or cobalt and the pH during the hydrogenation is set to a value between 7 and 10.

2. Process according to Claim 1, **characterized in that** the o-nitrophenoxy carbonyl compound of the general formula (II) has been purified by recrystallization before the hydrogenation.

3. Process according to Claim 1 or 2, **characterized in that** the pressure of the hydrogen gas is in the range between 10 and 400 bar.

4. Process according to any of Claims 1 to 3, **characterized in that** the temperature during the hydrogenation is in the range between 40 and 90°C.

5. Process according to any of Claims 1 to 4, **characterized in that** the hydrogenation is performed continuously with a mean residence time of the reactant solution in the catalyst suspension between 30 and 180 minutes, and a catalyst space velocity of 0.1 to 20 g of nitro compound per gram of catalyst and hour.

6. Process according to any of Claims 1 to 5, **characterized in that** the hydrogenation is performed in a solvent mixture of a C₁-C₃-alcohol and an aromatic solvent.

7. Process according to any of Claims 1 to 6, **characterized in that** the sponge metal catalyst is a sponge metal catalyst based on nickel with 0-15% by weight of iron.

8. Process according to any of Claims 1 to 6, **characterized in that** the sponge metal catalyst is a sponge metal catalyst based on nickel with 0-4% by weight of molybdenum.

9. Process according to any of Claims 1 to 6, **characterized in that** the sponge metal catalyst is a sponge metal catalyst based on cobalt with 0-5% by weight of aluminium.

## Revendications

1. Procédé pour la préparation de dérivés de benzomorpholine de formule générale (I) 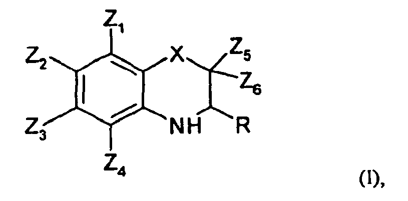 où
R représente hydrogène, un radical C₁-C₁₀-alkyle, phényle ou tétrahydrofurfuryle,
Z₁ à Z₆ représentent à chaque fois indépendamment hydrogène ou un radical C₁-C₁₀-alkyle et
X représente oxygène ou soufre,
par hydrogénation de composés d'o-nitrophénoxycarbonyle de formule générale (II) 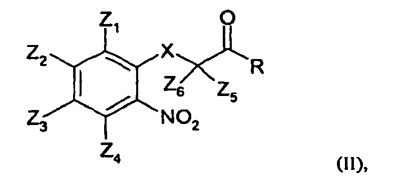 où
R, Z₁ à Z₆ et X ont la signification indiquée pour la formule (I),
**caractérisé en ce que** l'hydrogénation de composés de formule générale (II) est réalisée avec de l'hydrogène gazeux en présence d'un catalyseur de type éponge métallique à base de nickel ou de cobalt, dopé au molybdène et/ou au fer et/ou à l'aluminium et/ou au chrome et le pH est réglé pendant l'hydrogénation à une valeur entre 7 et 10.

2. Procédé selon la revendication 1, **caractérisé en ce que** le composé d'o-nitrophénoxycarbonyle de formule générale (II) a été purifié avant l'hydrogénation par recristallisation.

3. Procédé selon la revendication 1 ou 2, **caractérisé en ce que** la pression de l'hydrogène gazeux se situe dans la plage entre 10 et 400 bars.

4. Procédé selon l'une quelconque des revendications 1 à 3, **caractérisé en ce que** la température pendant l'hydrogénation se situe dans la plage entre 40°C et 90°C.

5. Procédé selon l'une quelconque des revendications 1 à 4, **caractérisé en ce que** l'hydrogénation est réalisée en continu à un temps de séjour moyen de la solution de produits de départ dans la suspension du catalyseur entre 30 et 180 minutes et à une charge du catalyseur de 0,1 à 20 g de composé nitro par gramme de catalyseur et par heure.

6. Procédé selon l'une quelconque des revendications 1 à 5, **caractérisé en ce que** l'hydrogénation est réalisée dans un mélange de solvants constitué par un C₁-C₃-alcool et un solvant aromatique.

7. Procédé selon l'une quelconque des revendications 1 à 6, **caractérisé en ce que** le catalyseur de type éponge métallique est un catalyseur de type éponge métallique à base de nickel contenant 0 à 15% en poids de fer.

8. Procédé selon l'une quelconque des revendications 1 à 6, **caractérisé en ce que** le catalyseur de type éponge métallique est un catalyseur de type éponge métallique à base de nickel contenant 0 à 4% en poids de molybdène.

9. Procédé selon l'une quelconque des revendications 1 à 6, **caractérisé en ce que** le catalyseur de type éponge métallique est un catalyseur de type éponge métallique à base de cobalt contenant 0 à 5% en poids d'aluminium.
